Europäisches Patentamt

European Patent Office

Office européen des brevets

(11) Numéro de publication: **0 094 887**
**B1**

## FASCICULE DE BREVET EUROPÉEN

(45) Date de publication du fascicule du brevet:
02.08.89

(21) Numéro de dépôt: 83400974.8

(22) Date de dépôt: 13.05.83

(51) Int. Cl.⁴: **C 12 N 15/00,** C 12 P 21/02,
C 12 N 1/20, A 61 K 39/205,
C 07 K 7/00 //
C12R1/125, C12R1/19

(54) **Nouveaux vecteurs d'expression de la protéine antigénique de la rage et leur application à la préparation de vaccins.**

(30) Priorité: 13.05.82 FR 8208401

(43) Date de publication de la demande:
23.11.83 Bulletin 83/47

(45) Mention de la délivrance du brevet:
02.08.89 Bulletin 89/31

(84) Etats contractants désignés:
AT BE CH DE FR GB IT LI LU NL SE

(56) Documents cité:
EP-A-0 041 767
FR-A-2 384 024

NATURE, vol. 294, 19.11.1981, A. ANILIONIS et al.:
"Structure of the glycoprotein gene in rabies
virus", pp. 275-278
DEV.CELL BIOL., vol 7, Elsevier North Holland, Inc.,
AMSTERDAM (NL), P.J. CURTIS et al.: "Cloning of
full length cDNA from the rabies virus glycoprotein
gene", pp. 721-725
BIOCHEMICAL AND BIOPHYSICAL RESEARCH
COMMUNICATIONS, vol. 103, no. 2, 30-11-1981
(US)m C.Y. LAI et al.: "Amino acid composition and
terminal sequence analysis of the rabies virus
glycoprotein: Identification of the reading frame
on the cDNA sequence", pp. 536-542
CHEMICAL ABSTRACTS, vol. 93, no. 9, 1er
septembre 1980, p. 200, résumé no. 90324f,

(73) Titulaire: TRANSGENE S.A., 16, rue Henri-
Regnault, F-92400 Courbevoie (FR)

(72) Inventeur: Kieny, Marie- Paule, 11 rue de
Gascogne, F-67000 Strasbourg (FR)
Inventeur: Lathe, Richard, 8, quai des Bateliers,
F-67000 Strasbourg (FR)
Inventeur: Lecocq, Jean- Pierre, 6, rue du Champs
du Feu, F-67116 Reichstett (FR)

(74) Mandataire: Warcoin, Jacques, Cabinet Régimbeau
26, avenue Kléber, F-75116 Paris (FR)

(56) Documents cité: (suite)
COLUMBUS OHIO (US), D. PENNICA et al. "In vitro
translation of rabies virus messenger RNAs"
NUCLEIC ACIDS RESEARCH, vol. 9, no. 2, 1981, IRL
Press Ltd., LONDRES (GB), J. MESSING et al.: "A
system for shotgun DNA sequencing", pp. 309-321
NATURE, vol. 294, no. 5939, novembre-décembre
1981 LONDRES (GB), "DNA sequencing vectors", p.
XXVII
SCIENCE, vol. 219, no. 4585, 11-02-1983,
WASHINGTON, D.C. (US), E. YELVERTON et al.:
"Rabies virus glycoprotein analogs: Biosynthesis in
Escherichia coli", pp. 614-620
GENE, vol. 19, 1982, Elsevier Biomedical Press
AMSTERDAM (NL); J. MESSING et al.: "A new pair
of M13 vectors for selecting either DNA strand of
double-digest resriction fragments, pages 269-276

## Description

La présente invention concerne plus particulièrement l'élaboration d'un vaccin contre la rage.

La rage est une maladie, provoquée par le virus rabique, qui se présente sous forme d'une encéphalomyélite gravissime qui peut être fatale en quelques jours.

La rage est une zoonose. Tous les animaux à sang chaud, qu'ils soient mammifères ou oiseaux, sont réceptifs. Le principal vecteur est en général constitué par des mammifères mordeurs, qu'ils soient domestiques ou sauvages.

Les principales victimes de la rage sont essentiellement l'homme et le bétail.

Les mesures de prévention efficaces ont permis de limiter les cas de rage humaine, qui se réduisent à quelques cas accidentels dans les pays fortement industrialisés.

Néanmoins, la rage reste encore une maladie très importante dans certains pays d'Amérique du Sud, en Afrique et en Asie.

L'arme essentielle de la lutte contre la rage est la prévention par la vaccination.

Les vaccins actuellement utilisés sont essentiellement constitués par des virus inactivés.

Toutefois, pour être reproduits industriellement, ces virus doivent être cultivés sur des milieux tels que des cerveaux de souriceaux nouveau-nés, sur des embryons d'oiseaux ou sur des cellules rénales animales ou sur des cellules diploïdes humaines, après quoi ils sont complètement inactivés par action de β-propiolactone par exemple.

Toutefois, les risques d'accidents neuroparalytiques liés à l'utilisation de ces vaccins ont grandement limité les possibilités de la vaccination, au moins au niveau humain.

En outre, bien que les nouveaux substrats utilisés pour la culture des virus aient beaucoup réduit le risque neurologique, celui-ci n'est pas complètement supprimé. De plus, l'utilisation de vaccins inactivés pose toujours le problème du risque d'une inactivation insuffisante. Enfin la nature particulière des milieux de culture conduit à de nombreuses difficultés au stade industriel qui font que le vaccin antirabique reste actuellement un vaccin très cher.

Le but de la présente invention est la préparation d'une protéine antigénique vaccinante exempte de risque d'accident neuroparalytique et qui, en putre, peut être préparée dans des conditions industrielles telles qu'elle assure un coût très faible du vaccin.

Le virus de la rage est un rhabdovirus.

Ce rhabdovirus est constitué d'une nucléocapside hélicoïdale contenant un ARN inclus dans une enveloppe lipoprotéinique ayant la forme d'un obus.

Ce virion comprend cinq protéines qui sont codées par le génome: G (glycoprotéine), N (nucléocapside), M1 et M2 (matrice) et L (large, transcriptase). Comme la couverture membranaire provient de l'hôte, le seul antigène efficace présent sur la surface est la glycoprotéine. Cette protéine transmembranaire a un poids moléculaire d'environ 75 000 daltons et possède une activité hémagglutinante.

La glycoprotéine purifiée est environ six fois plus active dans les essais d'hémagglutination que le virus intact.

Comme son nom l'indique, la glycoprotéine du virus de la rage est glycosylée, bien que le rôle de cette glycosylation soit inconnu.

La séquence d'ADN complémentaire du mARN de la glycoprotéine du virus de la rage a été décrite dans l'article de A. Anilionis et al., Nature 294; 275 - 276 (1981).

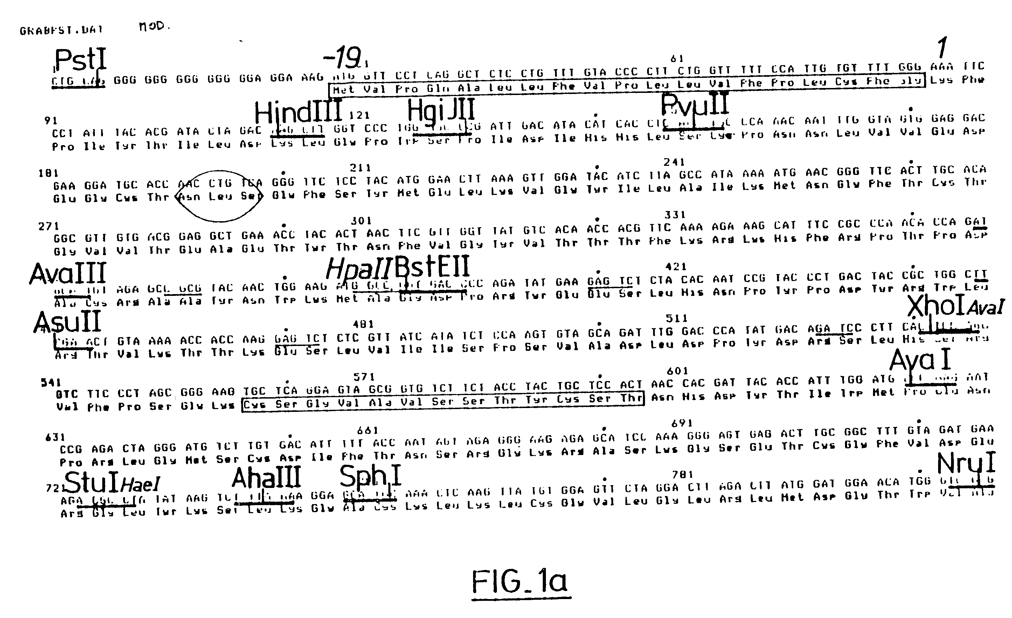

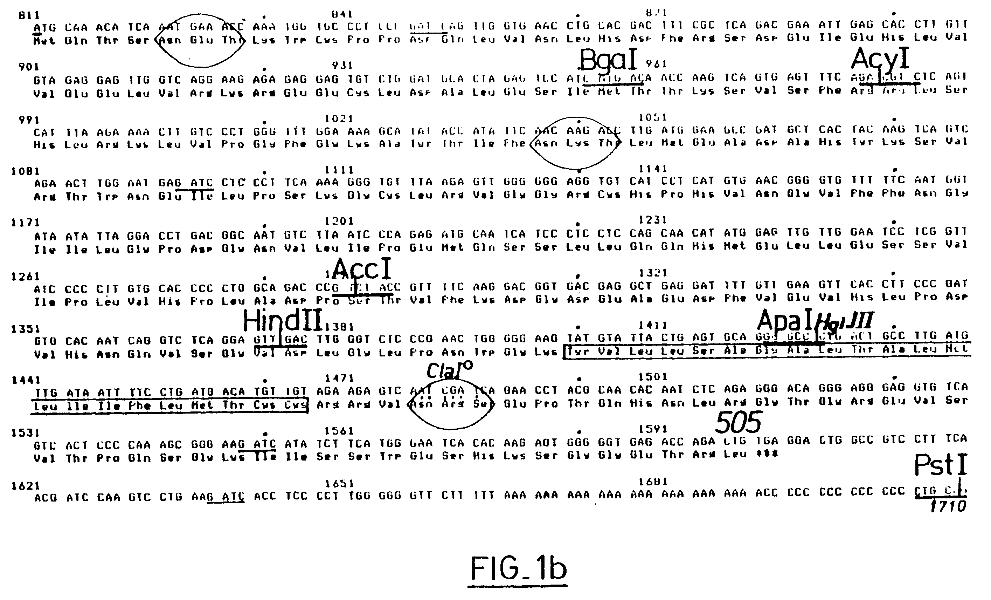

Ce gène a été utilisé dans le cadre des expérimentations qui seront décrites ci-après et est représenté schématiquement sur la figure 1 ci-annexée.

La présente invention concerne une bactérie exprimant une protéine antigénique de la rage, caractérisée en ce que ladite bactérie a été transformée ou transfectée par un vecteur d'expression qui comporte:

- la séquence d'ADN suivante:

    AGA GGC CTA TAT AAG TCT TTA AAA GGA GCA TGC AAA CTC AAG TTA
    TGT GGA GTT CTA GGA CTT AGA CTT ATG GAT GGA ACA TGG GTC GCG
- un promoteur de l'expression de cette séquence dans ladite bactérie.

Les vecteurs transformant ou transfectant les bactéries selon la présente invention peuvent, bien entendu, comporter d'autres fragments d'ADN destinés en particulier à assurer l'attachement des ribosomes correspondants ou bien à donner une configuration au vecteur facilitant l'expression de la protéine antigénique de la rage.

En particulier, ces vecteurs peuvent comporter tout ou partie de l'ADN d'un phage, notamment du phage M13, le promoteur du vecteur selon la présente invention étant d'ailleurs de préférence le promoteur de l'opéron lactose.

Parmi les phages M13 utilisables, il faut citer plus particulièrement les phages:

- M13tg102
- M13tg110
- M13tg111

- M13tg112
- M13tg115
- M13tg116
- M13tg117
- M13tg118
- M13tg120
- M13tg121

Il est bien entendu possible d'utiliser un autre type de promoteur comme par exemple un promoteur contrôlé tel que celui du bactériophage lambda et également d'introduire un site d'initiation de la traduction puissant tel que celui du gène cII du phage lambda.

Les bactéries selon l'invention peuvent également être transformées par des plasmides. En particulier, un plasmide vecteur qui comporte au moins:

- l'origine de réplication d'un plasmide bactérien,
- un promoteur du bactériophage λ: $P_L$, $P_R$ ou $P'_R$;
- une région codant pour l'initiation de la traduction.

La présence d'une origine de réplication pour un plasmide est essentielle pour permettre la réplication du vecteur dans les cellules bactériennes correspondantes, en particulier dans le cas de E. coli on utilisera, de préférence, l'origine de réplication du plasmide pBR322. Le plasmide pBR322 présente, en effet, l'avantage de donner un grand nombre de copies et ainsi d'augmenter la quantité de plasmides produisant la protéine désirée.

Il est, bien entendu, possible d'utiliser d'autres origines de réplication, en particulier celle d'un plasmide codant pour les facteurs de résistances quelconques ou d'un épison colicinogénique (c'est-à-dire conférant à une bactérie la faculté de synthétiser une colicine).

Toutefois, il convient de choisir des plasmides dont la réplication n'est pas contrôlée de façon trop sévère et qui ne présente pas de site de restriction indésirable, en dehors de la région de clonage.

Parmi les promoteurs du bactériophage λ, on utilisera, de préférence, le promoteur principal de gauche noté $\lambda P_L$. $P_L$ est un promoteur puissant responsable de la transcription précoce de λ.

Il est également possible d'utiliser d'autres promoteurs du bactériophage λ, notamment le promoteur de droite, $P_R$, ou le second promoteur de droite, $P'_R$.

Bien qu'il soit possible d'utiliser des séquences d'initiation de la traduction très variées, on préfère utiliser celle de la protéine cII du bactériophage λ qui sera nommée ci-après λcIIrbs.

Si l'utilisation de la région λcIIrbs est plus particulièrement intéressante, pour des raisons qui seront explicitées ci-après, il est néanmoins possible d'utiliser d'autres séquences d'initiation de la traduction, notamment celle du gène λE qui est limitée par des sites de restriction intéressants.

La région codant pour l'initiation de la traduction λcIIrbs est plus particulièrement intéressante car:

1°) il a été démontré que la protéine cII est synthétisée en grande quantité sous le contrôle du promoteur $P_L$;

2°) la région en cause est limitée par des sites de restriction qui permettent un isolement facile de ladite région;

3°) une enzyme de réstriction (NdeI) recouvre le codon d'initiation de la traduction.

Dans ces conditions, l'insertion de la séquence d'ADN (I) étrangers sur ce site avec reconstruction du codon d'initiation ATG permet l'expression de protéines non-fusionnées, c'est-à-dire ne contenant pas d'aminoacide étranger à la protéine que l'on devra préparer à l'exception de la méthionine initiale.

Par contre, le clonage de la séquence d'ADN (I) dans l'un des sites de restriction uniques en aval de λcIIrbs conduit à l'expression d'une protéine fusionnée.

La zone de clonage de l'ADN (I) est située en aval du promoteur $P_L$ et de la région d'initiation de traduction de cII. Parmi les sites d'insertion uniques qui figurent de préférence dans cette zone, on peut cite, EcoRI, SalI, AccI, BamHI, HindIII, HindII et PstI correspondant à des enzymes de restriction couramment utilisées. L'insertion des gènes codant pour une protéine particulière dans de tels sites placés à 40 - 50 bp en aval du codon d'initiation de la traduction conduit à la synthèse de protéines étrangères fusionnées à l'extrémité N terminale des aminoacides bactériens dérivant de cII.

Le vecteur en cause comporte, en outre, de préférence une fonction d'antiterminaison de transcription codée par exemple par le gène N de λ noté λN. En présence du produit de transcription du gène N la transcription à partir de $P_L$ se poursuit au delà de la plupart des signaux stop. Ceci écarte les problèmes posés par un arrêt prématuré de la transcription qui peuvent se produire lorsque les gènes étrangers clonés présentent de tels signaux stop. En outre, il a été démontré que l'expression à partir de $P_L$ est améliorée dans un environnement N⁺.

Lorsque le promoteur n'est pas $P_L$ ou $P_R$ mais $P'_R$ par exemple, la fonction d'antiterminaison peut être différente. $P'_R$ est responsable de la transcription des gènes tardifs du bactériophage λ. L'expression du gène tardif est régulée positivement par le produit du gène Q dont il a été démontré qu'il agit comme

3

antiterminateur de transcription analogue à la protéine N.

Q agit à la terminaison pour l'ARN 6S de λ constitutif initié à P'$_R$ en permettant ainsi la transcription de la région du gène tardif distal. L'expression de Q est elle-même contrôlée par P$_R$ qui, comme P$_L$, est régulé par le répresseur cl. Cette cascade d'interactions peut être reconstruite sur un plasmide d'expression et utilisée de la même façon pour P$_L$.

Afin d'écarter les problèmes de toxicité et d'instabilité du système hôte-vecteur en cas de production en continu de grandes quantités d'une protéine étrangère, il est nécessaire de prévoir le contrôle de l'activité du promoteur en lui adjoignant tout ou partie d'un système d'expression inductible, en particulier thermoinductible.

De préférence, le contrôle par la température de la synthèse de la protéine étrangère est effectué au niveau de la transcription au moyen d'un répresseur thermosensible codé dans la bactérie hôte, par exemple cl857, qui réprime l'activité de P$_L$ à 28°C mais qui est inactivé à 42°C. Le répresseur agit sur l'opérateur O$_L$ qui est adjacent au promoteur P$_L$. Bien que dans le cas précédent une partie du système d'expression thermoinductible soit partie intégrante de la bactérie hôte, il est possible de prévoir que ce système fasse partie du vecteur lui-même.

Le vecteur en cause peut également comporter un gène de résistance à un antibiotique, par exemple l'ampicilliné dans le cas de pBR322, mais d'autres gènes de résistance peuvent être utilisés, résistance à la tétracycline (Tet$^r$) ou au chloramphénicol (Cm$^r$).

L'incorporation d'un tel marqueur est nécessaire pour la sélection des bactéries contenant les transformants porteurs du plasmide selon l'invention pendant les expériences de clonage.

L'incorporation d'un gène de résistance permet d'augmenter la stabilité du plasmide en imposant une pression de sélection lors de la fermentation, et en outre facilite l'isolement des transformants.

Pour le clonage il est intéressant de disposer d'un système permettant de détecter l'insertion dans le plasmide de la séquence d'ADN (I).

A titre d'exemple, il est possible de prévoir dans la zone de clonage le fragment N-terminal de la β-galactosidase de E. coli (lacZ') en le fusionnant avec la région d'initiation de traduction dérivée de λcII, ce qui met la traduction du fragment α sous le contrôle des séquences de cII.

Le fragment α est complémenté par l'expression du fragment ω C-terminal codé dans l'hôte, ceci conduit à une activité β-galactosidase dans les cellules. Cette activité β-galactosidase produit des colonies bleues en présence d'un substrat chromophorique, le 5-bromo-4-chloro-3-indolyl-β-D-galactosidase.

A 28°C, le promoteur P$_L$ est inactivé, le fragment α n'est pas synthétisé et les colonies demeurent blanches. Lorsque la température est amenée à 42°C, le promoteur P$_L$ est activé, le fragment α est synthétisé et les colonies virent au bleu.

L'insertion d'ADN (I) dans les sites de clonage situés dans ce système de détection empêche la synthèse de la β-galactosidase et conduit donc à des colonies blanches aussi bien à 28°C qu'à 42°C.

Il est également possible de remplacer le gène lacZ' par d'autres gènes permettant une détection.

Parmi les souches bactériennes transformées par lesdits plasmides ou transfectées par lesdits phages selon la presente invention, il faut citer en particulier les bactéries gram négatives telles que Escherichia coli notamment lorsque l'ADN support est l'ADN du phage M13, mais d'autres souches bactériennes sont utilisables comme par exemple Bacillus subtilis.

La présente invention concerne également l'application de ces vecteurs à la préparation de glycoprotéines antigéniques de la rage par culture sur un milieu de culture approprié de souches bactériennes selon l'invention.

Bien entendu les conditions de culture et les milieux à utiliser ne constituent pas des caractéristiques du procédé selon la présente invention et peuvent être déterminés pour chaque souche par l'homme de métier.

La présente invention concerne également les protéines obtenues par la mise en oeuvre du procédé selon la présente invention et en particulier les protéines comportant la séquence suivante d'acide aminé:

$$\text{R G L Y K S L K G A C K L K L C G V L G L R L M D G T W V}$$

Les protéines ainsi obtenues immunoprécipitent avec les anticorps spécifiques correspondant à l'antigène majeur de la rage et peuvent donc être utilisées pour provoquer une immunostimulation permettant le traitement ou la prévention de la rage.

C'est pourquoi la présente invention concerne également des vaccins utiles aussi bien pour la prévention que pour le traitement de la rage commmportant à titre de principe actif immunostimulant au moins une protéine telle que décrite précédemment.

De préférence, la protéine antigénique selon la présente invention se présente sous forme d'une solution aqueuse lorsqu'elle doit être utilisée comme vaccin.

Bien entendu, la séquence d'acide aminé de cette protéine peut être synthétisée par voie chimique à l'aide de procédés connus.

De même, s'il est préférable que cette séquence soit incorporée dans une protéine plus importante, il n'est pas forcément indispensable que les acides aminés de part et d'autre correspondent à ceux du gène complet de la protéine antigénique de la rage, il est possible de prévoir d'autres acides aminés qui améliorent l'efficacité du composé, en particulier qui modifient sa solubilité. Il est même possible de prévoir que certains éléments différents des acides aminés soient greffés sur la chaîne.

Avant de décrire en détail les procédés mis en oeuvre pour la préparation des vecteurs selon la présente invention, il convient de résumer ci-après le schéma des manipulations correspondantes.

Les vecteurs selon la présente invention ont été préparés par insertion dans les sites appropriés de phages M13 de différentes séquences de restriction obtenues à partir du plasmide pRG décrit dans: A. Anilionis et al., Nature 294, 275 - 278 (1981).

La structure du gène codant pour la glycoprotéine de la rage est représentée à la figure 1.

Sur cette figure on a également représenté les différents sites de restriction présents sur ce gène ainsi que les acides aminés codés par cette séquence.

Les séquences de protéines encadrées correspondent aux régions hydrophobes de la glycoprotéine, quant aux régions entourées elles correspondent aux sites de glycosylation de la glycoprotéine.

Il semble que cette glycoprotéine soit traduite sous forme d'un précurseur comportant un peptide signal hydrophobe de 19 acides aminés, ce peptide est noté de 1 à - 19 sur la figure 1.

Il est intéressant de noter que la protéine contient deux autres régions hydrophobes, le peptide transmembranaire situé en fin de séquence, et une petite zone hydrophobe centrale dont la fonction est actuellement inconnue.

Comme on peut le remarquer sur la figure 1, le départ de la séquence qui code pour la glycoprotéine mature est suivi très rapidement par un site HindIII.

Il est donc intéressant de pouvoir cloner des fragments du gène débutant au site HindIII.

Afin de pouvoir cloner de façon satisfaisante une telle séquence d'ADN, il a été nécessaire de construire un vecteur avec un signal de traduction-initiation immédiatement suivi par un site HindIII en phase correcte.

A cette fin, on construit des vecteurs à partir du phage M13 dans lesquels le site HindIII est en phase 1, 2 ou 3 suivant les vecteurs afin d'être certain de l'expression correcte de la protéine.

Comme cela a été indiqué précédemment, et afin d'obtenir de préférence une protéine soluble, on essaiera d'éliminer la protéine transmembranaire située après le site HindII.

Par digestion du plasmide pRG avec les enzymes correspondantes, on isole les séquences de restriction suivantes:

1.  HindIII - PstI
2.  HindIII - XhoI
3.  HindIII - StuI
4.  HindIII - NruI
5.  HindIII - AosII
8.  StuI - PstI
10. HindIII - HindII

Ces séquences sont insérées dans les sites correspondants des phages M13: les fragments HindIII/ sont clonés dans le phage M13tg109 et le fragment StuI/PstI dans le phage M13mp701.

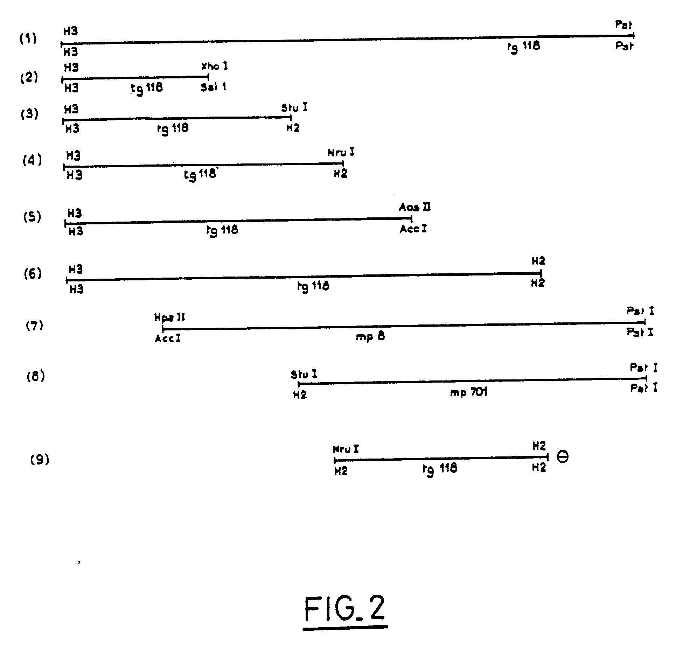

La figure 2 rassemble les différentes séquences de restriction qui ont été clonées, avec à la partie inférieure la mention du phage M13 utilisé ainsi que le site de restriction correspondant du phage.

La souche E. coli JM103 est infectée avec les phages comportant lesdites insertions et cultivée sur des milieux appropriés, en particulier supplémentés avec du succinate de sodium comme source de carbone de façon à limiter la répression catabolique du promoteur lac.

L'expression de ce promoteur est induite par addition de IPTG, la synthèse de la protéine étant suivie par incorporation de méthionine marquée.

Les bactéries sont ensuite collectées puis la glycoprotéine en est extraite et purifiée par incubation avec un antisérum correspondant à la protéine purifiée. Les complexes antigènes-anticorps sont récupérés par chromatographie d'affinité, lavés puis dissociés de façon à isoler la protéine antigénique.

Les poids moléculaires des protéines ainsi obtenues après purification sont cohérents avec ce qui était attendu, c'est-à-dire une protéine hybride comportant une partie β-galactosidase et une partie de la glycoprotéine antigénique de la rage correspondant à la séquence clonée.

Les résultats observés sont rassemblés dans le tableau I ci-après:

**Tableau I**

| Exp | Fragment cloné | Clone | Poids moléculaire des différentes parties de la protéine | | | Total | Poids moléculaire observé |
|---|---|---|---|---|---|---|---|
| | | | Peptide lac N-terminal | Polypeptide de la glycoprotéine centrale (k = kilodaltons) | Peptide C-terminal | | |
| 1 | HindIII-PstI | rg7 | 1,2 k | 54,5 k | - (a) | 55,7 k | 56,0 k |

| | | rg2 | | | | | |
|---|---|---|---|---|---|---|---|
| 2 | HindIII-XhoI | rg14 rg50 | 1,2 k | 15,5 k | 16,3 k[b] | 33,0 k | -[e] |
| 3 | HindIII-StuI | rg15 rg52 | 1,2 k | 22,4 k | 16,3 k[b] | 39,9 k | -[e] |
| 4 | HindIII-NruI | rg53 rg110 | 1,2 k | 25,4 k | 5,7 k[c] | 32,3 k | 31,0 k |
| 5 | HindIII-AosII (AcyI) | rg18 rg27 | 1,2 k | 31,8 k | 5,7 k[c] | 38,7 k | 38,5 k |
| 8 | StuI-PstI | rg123 rg124 | 1,2 k | 32,0 k | -[a] | 33,2 k | 25,0 k |
| 10 | HindIII-HindII (BamHI) | rg151 rg158 | 1,2 k | 46,2 k | 3,7 k[d] | 51,1 k | 51,0 k |

Les calculs ont été éffectués en admettant que le poids moléculaire moyen d'un acide aminé est de 110 daltons.

[a] la fin de la traduction se produit au codon stop de la glycoprotéine naturelle.
[b] la fin se produit au codon stop de l'α-peptide β-galactosidase.
[c] traduction hors phase du segment distal de l'α-peptide.
[d] traduction hors phase de la séquence distale codant pour la glycoprotéine.
[e] aucune bande n'a été observée lors de l'immunoprécipitation.

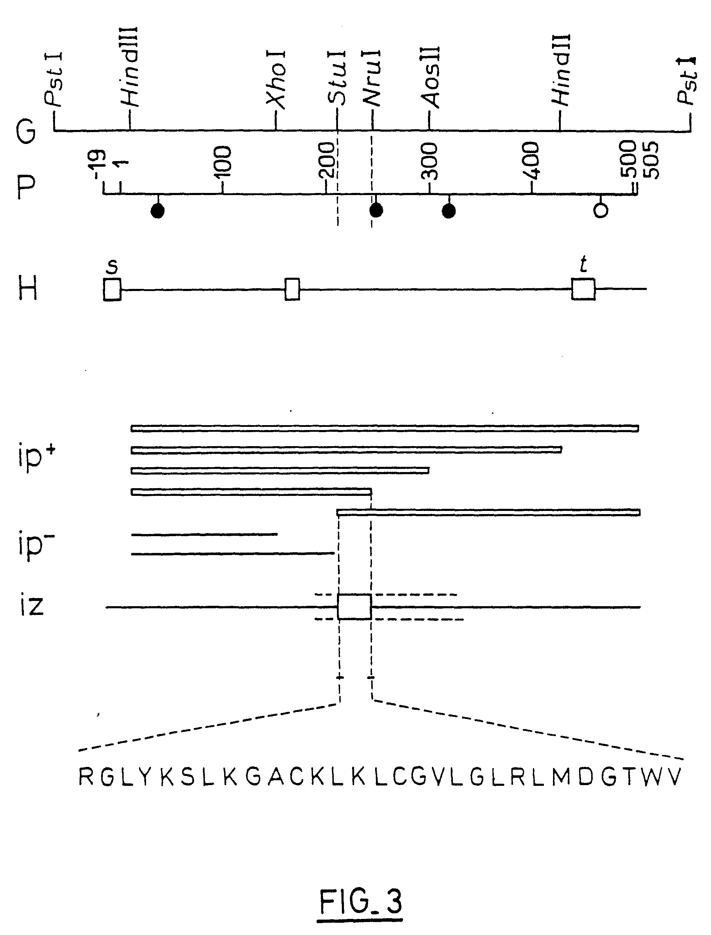

Ces résultats sont rappelés sur la figure 3 afin de mieux comprendre les enseignements que l'on peut tirer de ces expériences.

Sur cette figure 3 on a noté G le fragment de restriction PstI - PstI du gène de la rage sur lequel ont été représentés les principaux sites de restriction qui ont été mis en oeuvre.

On a ensuite représenté par P le schéma de la glycoprotéine antigénique de la rage, les chiffres représentant le nombre d'aminoacides comptés à partir de l'origine de la glycoprotéine mature.

On a représenté également par des cercles noirs ou blancs les sites de glycosylation.

Dans les mêmes conditions que pour P, on a représenté en H la séquence de la glycoprotéine antigénique sur laquelle figurent, représentées par des carrés, les régions hydrophobes.

On a ensuite schématisé par iP les différents fragments de restriction clonés. Les fragments de restriction notés iP+ et représentés en traits doubles correspondent à des fragments de restriction qui, après clonage, ont donné naissance à une protéine immunoprécipitante, par contre les fragments de restriction notés iP- et représentés par un seul trait n'ont pas donné lieu à une immunoprécipitation.

Les essais effectués sur iP+ et iP- conduisent donc par recouvrement à isoler une zone représentée dans le schéma iZ par un carré qui est la zone indispensable pour qu'apparaisse dans la protéine correspondante une propriété immunoprécipitante.

On a représenté sous iZ la protéine codée par ce fragment de recouvrement situé entre StuI et NruI.

Les abréviations utilisées dans cette description correspondent aux codes internationaux et en particulier les codes à une seule lettre pour les acides aminés sont les suivants:

R = Arg    C = Cys
G = Gly    V = Val
L = Leu    M = Met
Y = Tyr    D = Asp
K = Lys    T = Thr
S = Ser    W = Trp
A = Ala

Les exemples suivants sont destinés à mettre en évidence d'autres caractéristiques et avantages des procédés et des produits selon la présente invention; ils seront décrits en se référant aux figures annexées sur lesquelles:

La figure 1 représente la structure du gène codant pour la glycoprotéine de la rage;
La figure 2 représente les différentes séquences de restriction qui ont été clonées sur M13;
La figure 3 représente la stratégie pour mettre en évidence une séquence importante dans le cadre de l'invention;
La figure 4 représente la structure de différents phages dérivés de M13;
La figure 5 schématise la synthèse du plasmide pTG907;
La figure 6 schématise la synthèse du phage M13tg910;
La figure 7 schématise la structure du phage M13tg910;
La figure 8 schématise la synthèse du plasmide pTG908;

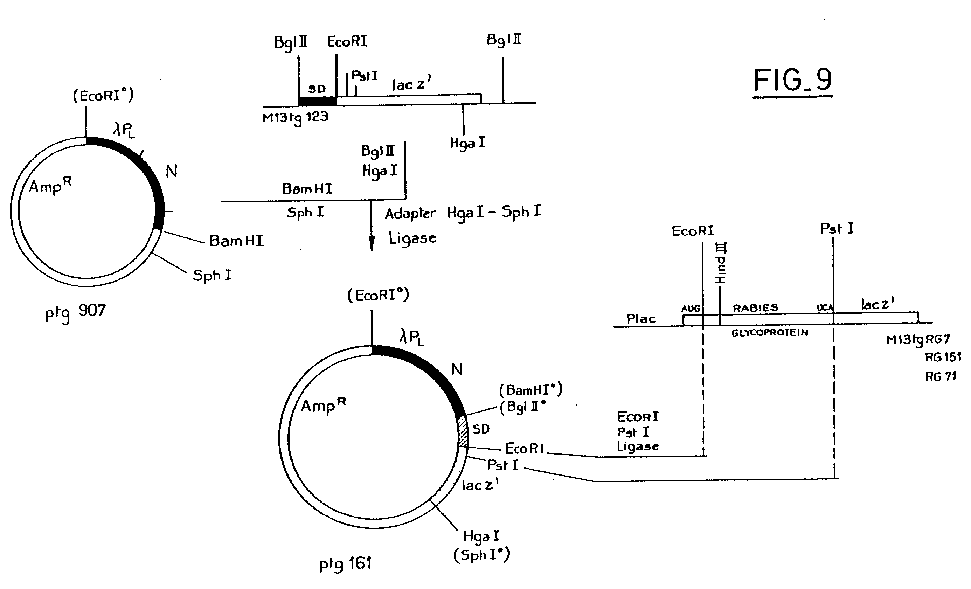

La figure 9 schématise la synthèse du plasmide pTG161;

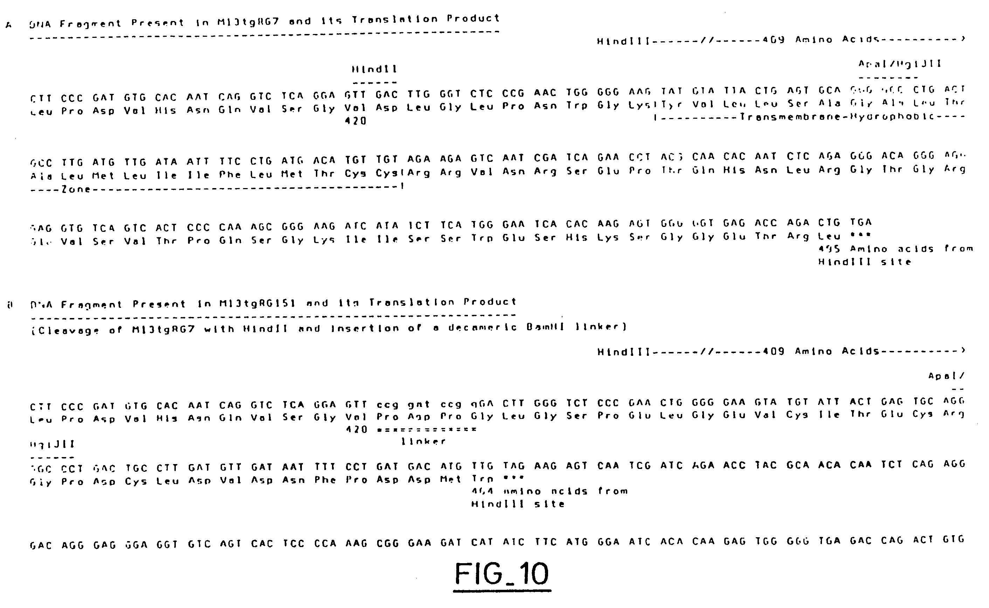

La figure 10 représente le fragment ADN présent dans les phages M13tgRG7 et M13RG151 et leur produit de traduction;

La figure 11 illustre la détection de la glycoprotéine de la rage produite par diverses souches selon l'invention;

La figure 12 représente l'effet de la température d'induction sur l'expression de la glycoprotéine de la rage.

En outre, sauf indication contraire les procédés et les souches mis en oeuvre sont les suivants:

a) Souches bactériennes

Les souches bactériennes utilisées dans le cadre de la présente invention sont les suivantes:

- TGE900 qui est une souche de E. coli ayant les caractéristiques suivantes: su⁻F⁻ his ilv bio (λcI857ΔBamΔHI)
- N6437, souche de E. coli ayant les caractéristiques suivantes: F⁻his ilv gal⁺ Δ8proC⁺: tn10 lacΔm15 (λcI857ΔBamΔHI).
- Jm103 qui est une souche de E. coli ayant les caractéristiques suivantes: Δ(lac-pro) supᴱ thi endA sbcB15 sttA rK⁻ nK⁺/F¹ traD36 proAB⁺ laciᵃ lacZΔm15.

Les souches mentionnées précédemment ont été utilisées parce qu'elles étaient disponibles, mais il est bien entendu qu'il est possible d'utiliser d'autres souches dans la mesure où elles présentent certaines caractéristiques essentielles qui sont rappelées dans le cours de la description détaillée.

b) Préparation des ADN

Des mini-préparations d'ADN de plasmides ou de phages M13 sont effectuées comme cela est décrit par Ish-Horowitz (référence 1) à la seule différence que l'ADN est précipité une seconde fois avec de l'éthanol avant d'être utilisé.

Les maxi-préparations sont effectuées comme cela est décrit dans la publication précédente, avec une purification complémentaire par un gradient de densité CsCl/bromure d'éthidium.

c) Techniques de clonages

Le traitement des ADN avec des enzymes de restriction est effectué, sauf indications contraires, en utilisant les conditions indiquées par le fabricant (New England Biolabs, Bethesda Research Laboratories et Boehringer Mannheim).

Lorsque cela est nécessaire, les phosphates des extrémités 5' sont éliminés en utilisant, soit une phosphatase alcaline bactérienne, soit une phosphatase d'intestin de veau, pendant 30 minutes à 37°C dans le tampon d'enzyme de restriction.

La réparation des extrémités cohésives utilisant la polymérase de Klenow (Boehringer Mannheim) est effectuée à 25°C pendant 15 minutes dans un mélange de 50 mMol. Tris HCl, pH 7,8, 5 mMol. MgCl₂, 10 mMol. de β-mercaptoéthanol, 0,4 mMol. de dNTps avec l'enzyme et 10 à 200 µg/ml d'ADN.

La nucléase S₁ (Miles) est utilisée à 2 u/µg d'ADN à 25°C pendant 30 minutes dans un milieu 0,3 Mol. NaCl, 0,03 Mol. NaOAc, pH 4,8, 0,003 Mol. ZnCl₂.

Ba131 est utilisée selon le procédé de Panayotatos et al. (référence 2). Les ligations sont effectuées à 15°C (sauf lorsque cela est indiqué différemment) pendant 4 à 24 heures en utilisant de l'ADN ligase T₄ (Boehringer Mannheim) avec 100 mMol. de NaCl, 66 mMol. de Tris HCl, pH 7,5, 10 mMol. de MgCl₂, 0,5 mMol. de spermidine, 0,2 mMol. de EDTA, 2 mMol. de DTT, 1 mMol. d'ATP, 0,1 mg/ml de BSA et 5 à 50 µg/ml d'ADN.

Pour la ligation d'extrémités cohésives, on utilise environ 30 unités/ml de ligase. Pour la ligation des extrémités franches on utilise environ 100 unités/ml de ligase.

Entre les différentes réactions enzymatiques, des échantillons d'ADN sont extraits avec un mélange phénol/chloroforme puis précipités à l'éthanol. Lorsque cela est nécessaire, du tARN de E. coli ou de levures est utilisé comme entraîneur. Les adaptateurs moléculaires (Collaborative Research, Bethesda Research Laboratories, New England Biolabs) sont préhybridés et utilisés en excès molaire de 10 à 50 fois pour les extrémités franches d'ADN utilisant les conditions de tampon décrites précédemment avec 100 unités/ml de ligase T₄ à 4°C pendant 15 heures. Lorsque l'on utilise des adaptateurs non phosphorylés, les adaptateurs qui n'ont pas réagi sont éliminés directement après ligation par précipitation avec le tétrachlorhydrate de spermine (Hoopes et al. (référence 3).

Lorsque l'on utilise des adaptateurs phosphorylés, le mélange de ligation est tout d'abord extrait avec un mélange phénol/chloroforme puis précipité avec de l'éthanol avant coupure spécifique avec les enzymes de restriction appropriées puis précipitation avec le tétrachlorhydrate de spermine.

Les cellules bactériennes compétentes sont préparées puis transformées avec les plasmides ou transfectées par l'ADN de M13 selon les procédés décrits par Dagert et Ehrlich (référence 4).

**Exemple 1**

**Construction de nouveaux vecteurs M13**

La construction de ces vecteurs est indiquée dans le tableau II ci-après sur lequel figurent les références du phage de départ, la nature de l'enzyme de restriction avec laquelle il a été découpé, le traitement particulier qu'ont subi les fragments ainsi obtenus et la nature de l'insert qui a été fixé dans les sites ainsi révélés.

Le phage M13mp7 est commercialisé par la société BRL.

M13mp701 est obtenu à partir de M13mp7 par remplacement du fragment PstI - EcoRI à droite (CTG CAG ... GAA TTC) par la séquence: CTG CAG CAA TTC.

Le principe de la construction est décrit dans le schéma suivant de préparation de M13tg118 à partir de M13mp701:

```
ATG ACC ATG ATT ACG AAT TCC CCG GAT CCG TCG ACC TGC AGC AAT TCA CTG GCC          M13=p701

                              Bam HI  ↓

ATGACCATGATTACGAATTCCCCG          GATCCGTCGACCTGCAGCAATTCACTGGCC
TACTGGTACTAATGCTTAAGGGGGCCTAG          GCAGCTGGACGTCGTTAAGTGACCGG

                              DNA polymerase  ↓

ATGACCATGATTACGAATTCCCCGGATC          GATCCGTCGACCTGCAGCAATTCACTGGCC
TACTGGTACTAATGCTTAAGGGGGCCTAG          CTAGGCAGCTGGACGTCGTTAAGTGACCGG

                              Linker HindIII  ↓

ATGACCATGATTACGAATTCCCCGGATCCCAAGCTTCG          CCAAGCTTGGGATCCGTCGACCTGCAGCAATTCACTGGCC
TACTGGTACTAATGCTTAAGGGGGCCTAGGGTTCGAACC          GGTTCGAACCCTAGGCAGCTGGACGTCGTTAAGTGACCGG

                              HindIII  ↓

ATGACCATGATTACGAATTCCCCGGATCCCA          AGCTTGGGATCCGTCGACCTGCAGCAATTCACTGGCC
TACTGGTACTAATGCTTAAGGGGGCCTAGGGTTCGA          ACCCTAGGCAGCTGGACGTCGTTAAGTGACCGG

                              T4 DNA ligase  ↓

ATG ACC ATG ATT ACG AAT TCC CCG GAT CCC AAG CTT GGG ATC CGT CGA CCT GCA GCA ATT CAC TGG CC.
    --------   --------   -------   --------  --------   --------
    Eco RI     Bam HI    Hind III  Bam HI  Sal I    PstI
```

**Tableau II**

| Nom | Phage parental | Site de clivage | Traitement | Insert | Phase de HindIII | Phase de BamHI | Phase de PstI | Complémentation de lac α +/- |
|---|---|---|---|---|---|---|---|---|
| M13mp701 | M13mp7 | Construction D.R. Bentley | | | O | I | I | + |
| M13tg102 | M13mp701 | AccI | ADN pol. | séquence HindIII(a) | I | I | I | + |
| M13tg110 | M13mp701 | AccI | S₁nucléase | séquence HindIII(a) | III | I | II | - |
| M13tg111 | M13tg102 | BamHI | ADN pol. | séquence HindIII(a) | III | O | III | - |
| M13tg112 | M13tg102 | BamHI | ADN pol. | séquence HindIII(b) | II | O | II | - |
| M13tg115 | M13tg110 | EcoRI | S₁nucléase | séquence BglII(c) | II | III | I | + |
| M13tg116 | M13tg110 | HindIII-PstI | - | HindIII-PstI(d) | III | I | I | + |
| M13tg117 | M13tg110 | EcoRI-BamHI | - | EcoRI-BamHI(e) | II | III | I | + |
| M13tg118 | M13mp701 | BamHI | ADN pol. | séquence HindIII(a) | III | II | II | - |
| M13tg120 | M13mp7 | EcoRI-PstI | S₁nucléase ADB pol. | adaptateur GATCTOCA(f) | O | I | I | + |
| M13tg121 | M13tg120 | EcoRI-PstI | - | Fragment EcoRI-PstI de tg102 | I | I | I | + |

(a) adaptateur de séquence HindIII phosphorylée CCAAGCTTGG
(b) délétion spontanée d'une base avant l'adaptateur HindIII
(c) adaptateur de séquence BglII non phosphorylée CAGATCTG
(d) fragment HindIII-PstI de M13mp8
(e) fragment EcoRI - BamHI de M13mp
(f) ligation de l'adaptateur dans le site Pst1, remplissage par A, traitement par la nucléase S₁

Les protocoles d'utilisation des enzymes (restriction DNA polymérase, T₄DNA ligase) sont ceux indiqués dans les notices des fournisseurs. Le linker HindIII est un oligonucléotide synthétique de séquence 5'pCCAAGCTTGG3' (Collaborative Research. Inc., Waltham, Mass 02154, U.S.A.). La séquence du phage recombinant M13tg118 obtenu a été vérifiée. Le DNA double brin des phages M13tg118 et mp701 a été purifié selon la méthode de Ish-Horowicz D. et Burke J.F. (Nucl. Acids Res. 9. 2989 - 2998. (1981)), coupé avec les enzymes de restriction appropriées et déphosphorylé à l'aide de phosphatase alcaline intestinale de veau (Sigma).

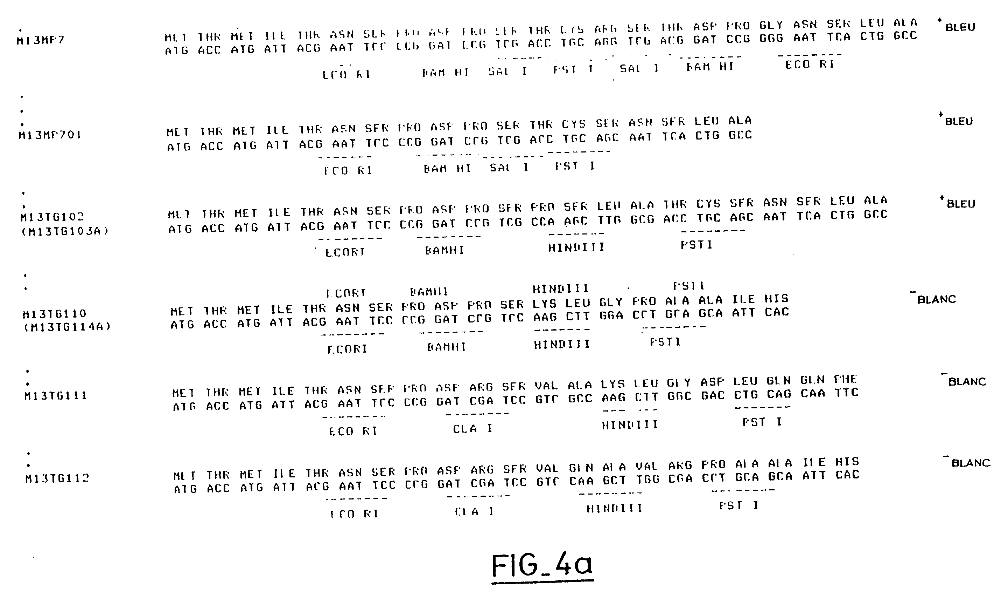

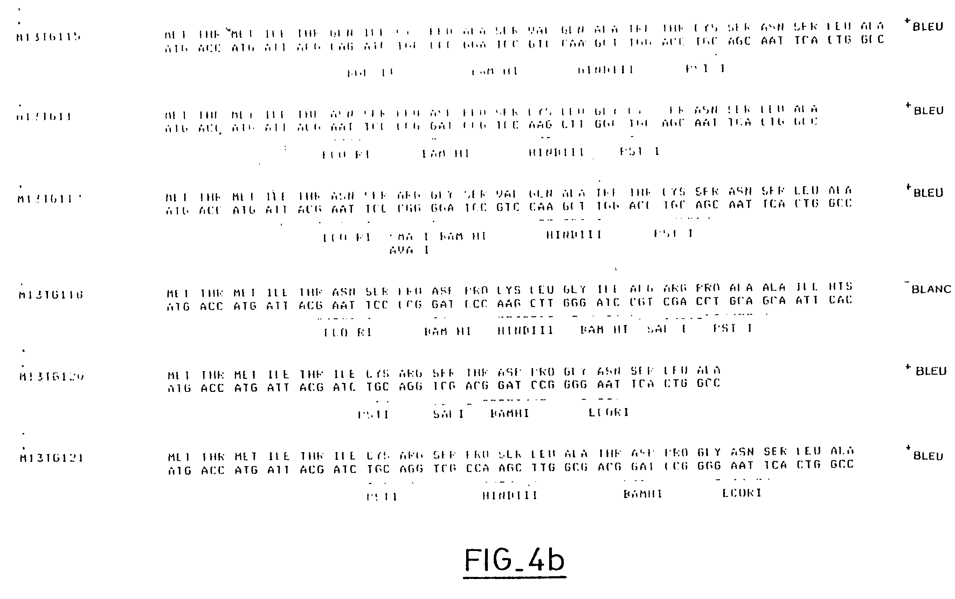

La figure 4 représente les différents phages M13 en cause ainsi que les phages mp7 et mp701 qui ont servi à les élaborer.

Sur la figure 4, on a représenté, outre la séquence d'ADN du phage, la protéine correspondante ainsi que les sites de restriction.

Sur la droite de la figure on a également fait figurer l'existence ou non d'une α-complémentation pour le gène lac lorsque le phage M13 est inséré dans un E. coli, en particulier dans la souche JM103.

## Exemple 2

### Préparation des fragments du gène de la glycoprotéine de la rage

i. Purification du fragment de 1,6 Kb HindIII-PstI

Le plasmide pRG, inclus dans la souche de E. coli HB101, a été purifié selon la méthode de lysat clair de Ish-Horowicz. 500 µg de DNA plasmidial a été coupé par les enzymes de restriction HindIII et PstI. Le produit de digest a été déposé sur un gel de "low temperature" agarose (BRL). Après migration, le morceau de gel contenant la bande de 1,6 Kb a été découpé, dissout à 67°C, phénolisé et l'ADN précipité à l'éthanol et appliqué sur une colonne de 1 ml de Sephadex G-50 (Pharmacia) équilibré au TE (Tris/HCl pH 7,5 10 mM; EDTA 1 mM). Les fractions exclues ont été précipitées à l'éthanol (2 vol.) et reprises dans 100 µl de tampon TE. 200 µg de DNA purifié ont été ainsi obtenus.

ii. Digestion par des enzymes de restriction

Pour le clonage de chaque sous fragment, 5 µg de DNA purifié ont été digérés par les enzymes de restriction appropriées.

## Exemple 3

### Clonage des sous fragments

Les sous fragments du gène de la glycoprotéine de la rage ont été ligués dans les vecteurs préparés comme indiqué précédemment. La ligation a été effectuée mole à mole, dans les conditions préconisées par le fournisseur.

Le produit de ligatiom a ensuite été transformé dans la bactérie E. coli JM103. La présence et la taille des inserts dans les phages M13 obtenus ont été testés par la méthode de mini lysat de Birnboim H.C. et Doly J. (Nucl. Acids Res. 7, 1513 - 1523 (1979)). Des phages recombimants ont été obtenus pour les fragments

HindIII-PstI
HindIII-XhoI
HindIII-StuI
HindIII-NruI
HindIII-AosII
HindIII-HindII
StuI-PstI

## Exemple 4

### Immunoprécipitation des protéines synthétisées

Les phages recombinants ont été cultivés dans 10 ml de milieu minimum $M_9$ (Miller, J.H, Experiments in Molecular Genetics** (1972)) avec du succinate de sodium (0,5 %) comme source de carbone, jusqu'à une $DO_{650}$ de 0,25. A ce stade, de l'IPTG* a été ajouté à la concentration de 0,1 mM, ainsi que de la L-($^{35}$S)-méthionine (12 µCi/ml) et de la L-méthionine à la concentration de 5 µg/ml. Après 1 h d'incorporation, les bactéries ont été récoltées et lysées en présence de lysozyme à la concentration de 2 mg/ml pendant 5 mm à 4°C. Le surnageant clarifié a été incubé à 4°C pendant 16 h avec des anticorps antiglycoprotéine rabique (fournis par l'Institut Wistar, U.S.A.) puis 1 h à 25°C avec 8 mg de protéine A Sepharose (Pharmacia) selon Lathe R. et al., Nature 284, 473 - 474 (1980). Après des lavages abondants, cette protéine A a été déposée sur un gel d'acrylamide 10 % + SDS classique (Laemmli U.K. Nature 227, 680 - 685 (1970)). Après migration, le gel a été traité au PPO, puis séché et mis à exposer (Bonner W.M. et Laskey R.A. Eur. J. Biochem. 46, 83 - 88 (1974)).

Ces essais mettent en évidence que la glycoprotéine synthétisée par les bactéries ainsi que les sous fragments sont efficacement reconnus par l'antisérum en particulier pour les fragments codés par les séquences HindIII/PstI, HindIII/Acyl, StuI/PstI et HindIII/NruI.

* IpTG: Isopropyl thiogalactopyranoside
** Gold Spring Harbor Laboratory

## Exemple 5

La préparation d'un plasmide selon l'invention implique tout d'abord la préparation d'un plasmide comportant:

- l'origine de réplication de PBR322,
- le gène de résistance à l'ampicilline de ce même plasmide amp$^R$,
- le promoteur $P_L$ et le gène λN,

et, en outre, au cours de cette synthèse on doit s'efforcer de faire disparaître les sites de restriction gênants afin de pouvoir obtenir plus tard dans le cours de la synthèse des sites uniques de restriction.

### 1) Suppression du site PstI dans pBR322

Le plasmide de base utilisé est le plasmide pBR322; toutefois, celui-ci présente l'inconvénient d'avoir à l'intérieur du gène amp^R un site de restriction PstI, car un site de même nature sera utilisé par la suite dans la zone de clonage comme site unique de restriction. Il convient donc de faire disparaître ce site de restriction PstI en utilisant un mutant du plasmide pBR322, le plasmide pUC8, dans lequel le gène de résistance à l'ampicilline ne présente pas de site de restriction PstI (ce site a été éliminé par mutation in vitro). pBR322 est commercialisé notamment par Bethesda Research Laboratories et pUC8 est décrit dans l'article référencé 5.

Pour ce faire, on échange le fragment PvuI/PvuII de 1 669 bp de pBR322 avec le fragment analogue PvuI/PvuII du plasmide pUC8. Afin de réaliser cet échange les plasmides pBR322 et pUC8 sont traités successivement par PvuI, PvuII, puis circularisés par section d'une ligase.

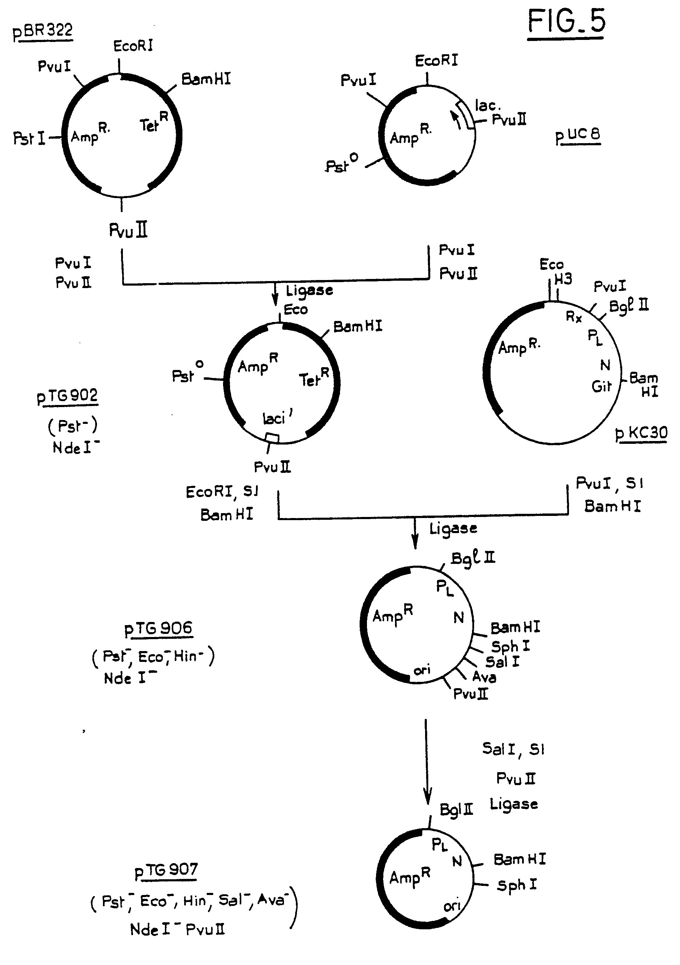

On obtient ainsi le plasmide pTG902 qui ne présente plus de site de restriction PstI et qui a également perdu le site de restriction NdeI présent à l'origine sur pBR322 (non représenté sur la fig. 5). En outre, le plasmide pTG902 porte un fragment de 50 bp correspondant à la séquence laci' dans lequel se trouve le site PvuII.

## 2) Insertion du promoteur P_L et du gène λN et préparation du plasmide de pTG907

Le promoteur P_L et le gène λN sont isolés du plasmide pKC30 pour être insérés dans pTG902, le segment prélevé contient également l'opérateur O_L sur lequel agira le répresseur thermosensible cI850, comme cela sera décrit dans les essais. En outre, le procédé permet de supprimer les sites EcoRI et HindIII tout en conservant un site unique BamHI en aval du gène N pour pouvoir ensuite insérer λcIIrbs. Le plasmide de pKC30 est décrit dans la référence 6.

pTG902 est coupé en son site de restriction unique EcoRI et les extrémités 5' sortantes sont éliminées par traitement à la nucléase S_1. Après une digestion avec BamHI, le fragment le plus important est purifié sur gel.

Le fragment portant le promoteur P_L et le gène λN est préparé de la même façon à partir de pKC30 en traitant successivement le plasmide par PvuI, la nucléase S_1 et BamHI. Les fragments, après purification sur gel, sont soumis à l'action de la ligase, ce qui conduit à la fusion EcoRI/PvuI et à la reconstitution du site BamHI.

Le mélange de ligation est utilisé pour transformer des cellules hôtes compétentes, TGE900, à 30°C. Cette souche contient le prophage λ délété, λcI857ΔBamΔHI, qui fournit le répresseur de λ thermosensible, cI857, qui est nécessaire pour bloquer la transcription à partir de P_L.

Ceci est important car l'activité de P_L dans un milieu N+ est léthale compte tenu de la fonction d'antiterminaison de N.

Après analyse par enzymes de restriction, les clones contiennent des plasmides de structure correcte et sont ensuite testés pour leur manque de viabilité à 42°C.

L'un des plasmides obtenus, pTG906, est traité de façon à éliminer le segment PvuII-SalI de manière à supprimer les sites de restriction compris sur ce segment et afin de faire disparaître également les deux sites de restriction extrêmes. Pour ce faire, pTG906 est traité successivement avec SalI, la nucléase S_1, PvuII et la ligase.

On obtient ainsi le plasmide pTG907 qui comporte l'ensemble des éléments évoqués au début de cette étape et, en outre, qui est Pst- Eco- Hind- Sal- Ava- Nde- PvuII-. La synthèse de ce plasmide est représentée sur la figure 1.

## 3) Clonage de la région λcIIrbs

La seconde phase importante de la synthèse consiste à insérer la région λcIIrbs sous forme d'un fragment Aval/TaqI dans le début du gène lacZ' (fragment α de la β-galactosidase) lequel a été cloné dans le phage M13 nommé M13tg110. Cette stratégie permet un test fonctionnel simple pour rbs, à savoir la production de la protéine lacZ' et, par conséquent, d'obtenir des plaques bleues en présence d'IPTG et de Xgal; ceci permet également un séquençage rapide de la construction en utilisant la méthode dite du didéoxy.

La région cIIrbs est isolée à partir d'un plasmide pOG11 qui contient un fragment de λ.HaeIII/Sau3A qui s'étend du milieu du gène cro jusqu'au milieu de la région codant pour cII (cro et cII étant impliqués notamment dans la régulation de la lysogénie du bactériophage λ).

On prélève dans pOG11 le fragment Aval/TaqI de 186 bp contenant la région cIIrbs et on le traite avec la polymérase Klenow. D'autre part, on traite le phage M13tg110 par BamHI puis par la polymérase de Klenow suivi d'un traitement à la phosphatase intestinale de veau. Les fragments obtenus sont soumis à l'action de la ligase T_4.

Un examen des séquences du fragment de pOG11 comportant la région cIIrbs et du phage M13tg110 permet de prévoir que l'insertion du fragment portant cIIrbs dans le site BamHI de M13tg110 doit conduire à l'obtention de plaques bleues après fermentation des bactéries transfectées compte tenu du fait que le gène lacZ' est en phase pour la traduction à partir de AUG du gène de cII, tandis que les plaques correspondant à la souche parentale M13tg110 sont blanches.

Les plaques bleues sont prélevées puis les colonies sont sélectionnées par analyse de restriction enzymatique des mini-préparations et l'on vérifie ensuite par séquençage que la construction obtenue est correcte.

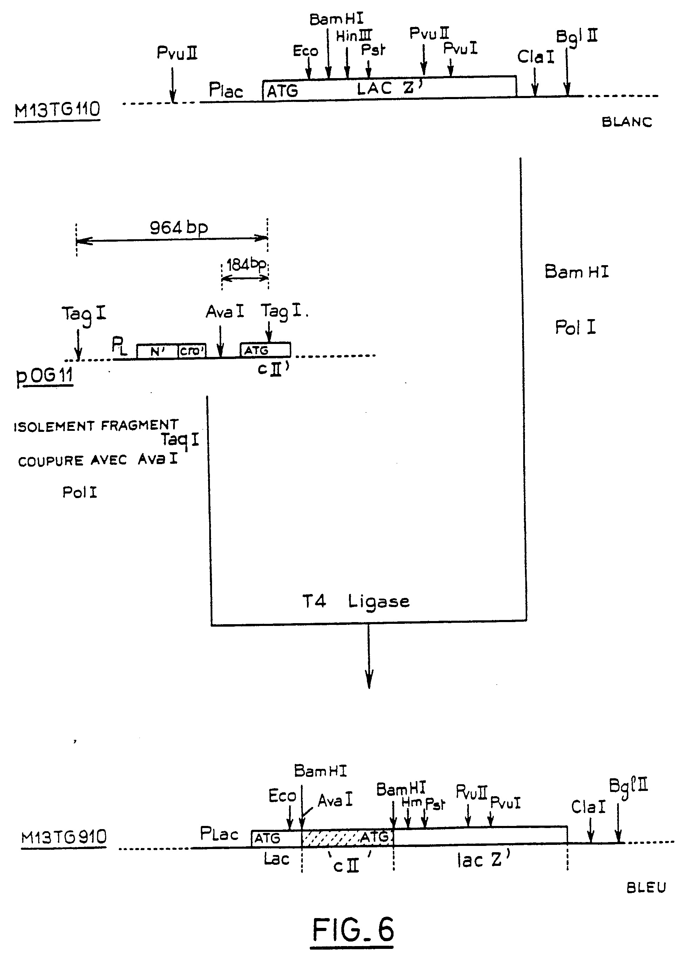

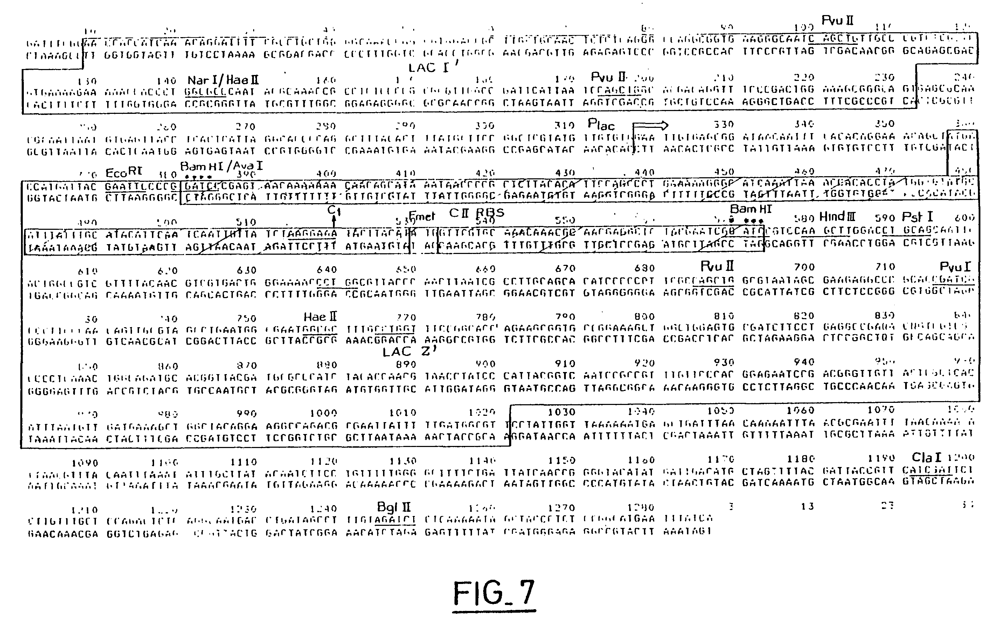

On obtient ainsi un clone résultant M13tg910 dont la structure globale est représentée à la partie inférieure de la Figure 6 et la structure détaillée est représentée à la figure 7.

On constate que l'insertion du fragment cIIrbs reconstruit en amont les sites BamHI et Aval et en aval le site

BamHI.

La traduction à partir de AUG de cII conduit à la fusion des 13 aminoacides terminaux de cII aux 8 aminoacides du NH$_2$ terminal de la protéine lacZ'.

#### 4) Insertion du fragment λcIIrbs dans le plasmide pTG907

La troisième étape de cette synthèse consiste à transférer le fragment cIIrbs/lacZ' du phage M13tg910 sur le plasmide vecteur PTG907 préparé précédemment.

Pour ce faire, il convient tout d'abord d'éliminer les sites EcoRI, BamHI et AvaI en amont de cIIrbs puis d'insérer un site BgIIII.

Dans ces conditions, cIIrbs peut être prélevé sous forme d'un fragment BgIII-BgIII et placé dans le site BamHI en aval du promoteur P$_L$ et du gène λN de pTG907.

Le phage M13tg910 est digéré avec EcoRI puis traité avec Ba131 puis ensuite par la polymérase de Klenow. Les fragments obtenus sont alors soumis à l'action de la ligase en présence d'adaptateurs BgIII non phosphorylés. Le mélange de ligation obtenu est utilisé pour transformer des cellules compétentes JM103.

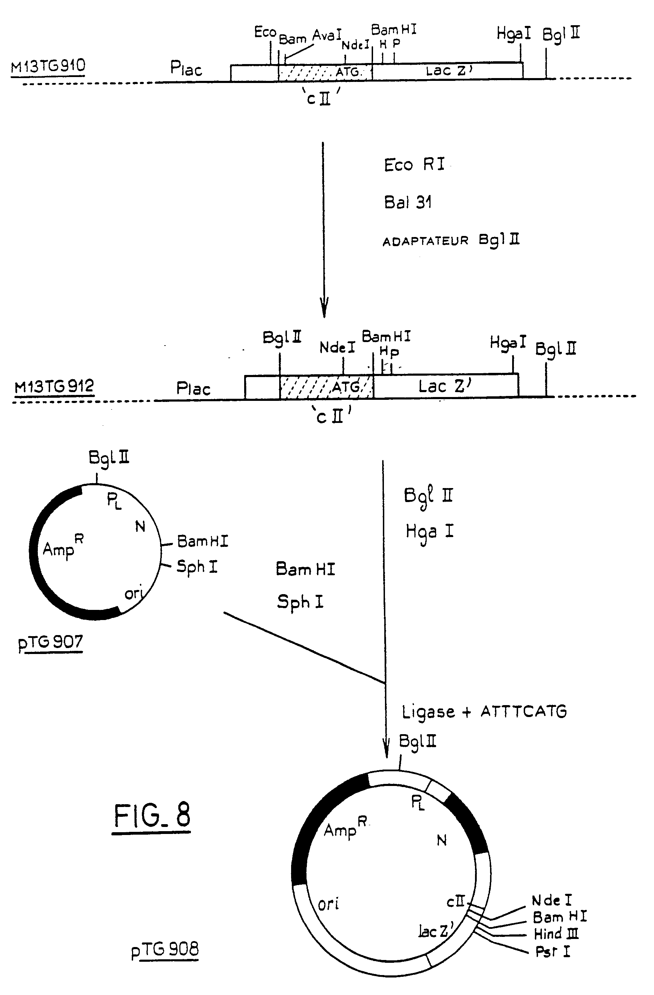

On sélectionne alors les plages bleues. Ces clones sont ensuite analysés afin de vérifier qu'ils contiennent le site BgIII et qu'ils ne présentent plus de site EcoRI ou BamHI en amont. On obtient ainsi des clones tels que M13tg912 dont la structure est représentée sur la Figure 8.

Le traitement par Ba131 a produit une délétion de 101 bp éliminant les sites EcoRI, BamHI et AvaI; ainsi que les séquences de lac ATG et lac Shine/Dalgarno. Le site BgIII introduit se trouve placé environ 100 bp en amont de l'ATG de cII et 10 bp en aval de P$_{lac}$.

Afin de remplacer le site BamHI du phage M13tg912 par un site EcoRI, ce phage est traité par BamHI, la nucléase S$_1$ puis le produit de ces traitements est ligué en présence d'un adaptateur EcoRI GGAATTCC, on obtient ainsi le phage M13tg913.

La suite de la synthèse met en oeuvre un adaptateur HgaI/SphI qui permet l'insertion d'un fragment BgIII/HgaI portant cIIrbs et lacZ' entre les sites BamHI et SphI de pTG907.

Le fragment BgIII/HgaI de M13tg913 est introduit entre les sites BamHI et SphI de pTG907 grâce à un adaptateur SphI-HgaI, afin de générer le plasmide pTG161.

Afin d'avoir une première indication montrant que les différents éléments du système d'expression se conduisent comme cela est désiré, le plasmide obtenu, pTG161, peut être transféré dans une souche hôte N6437. Cette souche qui possède à la fois c1857 et le fragment ω de la β-galactosidase complémentant le fragment, qui est codé par le plasmide après transformation permet d'observer sur une boîte contenant IPTG + Xgal le passage de la couleur blanche à 28°C au bleu environ 30 minutes après lorsqu'on la transfère à 42°C.

### Exemple 6

Les différents fragments du gène de la rage sont clonés dans le plasmide pTG161 sous forme de fragment EcoRI/PstI par digestion du plasmide et du phage M13 correspondant avec ces enzymes. Les fragments de restriction sont liés par la ligase T$_4$.

On obtient de cette façon divers plasmides:

pRG107, à partir du phage M13rg7
pRG71-22 à partir d'un mutant du phage M13rg7
pRG72 et 73, à partir de phages dérivés de M13rg71-22
pRG151, à partir du phage M13rg151
pRG123, à partir du phage M13rg123

Ces plasmides sont utilisés pour transformer la souche de E. coli TGE900.

Les souches TGE900 incorporant les différents plasmides sont mises en culture sur un milieu riche à 28,5°C jusqu'à une densité optique de 0,1 à 660 nm, puis de nouveau incubées pendant 5 heures à 35°C. Les cellules sont récupérées par centrifugation et soumises à une électrophorèse sur gel en présence de SDS.

Les protéines séparées sur gel sont transférées par électrophorèse sur de la nitrocellulose. Les antigènes capables de se lier aux anticorps monoclonaux dirigés contre les glycoprotéines de la rage sont détectés par incubations successives avec les anticorps et un antisérum de chèvre marqué à 1125 dirigés contre une immunoglobuline de souris, comme cela est décrit par Burnette (1981).

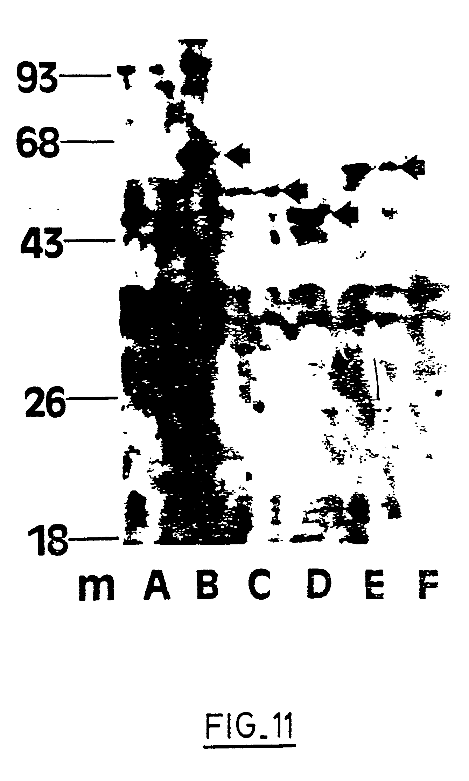

Les bandes radio-actives sont révélées par autoradiographie (figure 11). Les différents résultats correspondent aux colonnes suivantes:

A - plasmide pTG161 sans insertion
B - plasmide pRG7
C - plasmide pRG71-22
D - plasmide pRG73
E - plasmide PRG151

EP 0 094 887 B1

F - plasmide pRG123
m - indique la position des marqueurs de poids moléculaire en kD

Compte tenu de la nature de ces expériences, les positions observées sont seulement approximatives.

La position des bandes spécifiquement détectées sont indiquées par des flèches. Dans la colonne F une bande légère à environ 38 kD peut indiquer une réaction légère de l'anticorps avec la séquence de glycoprotéines rabiques exprimées dans cette souche.

La fixation initiale des antigènes rabiques est effectuée en utilisant un mélange de deux anticorps monoclonaux 101-1 et 509-6 neutralisant le virus qui a été fourni par le Wistar Institute.

## Exemple 7

### Influence de la température d'induction

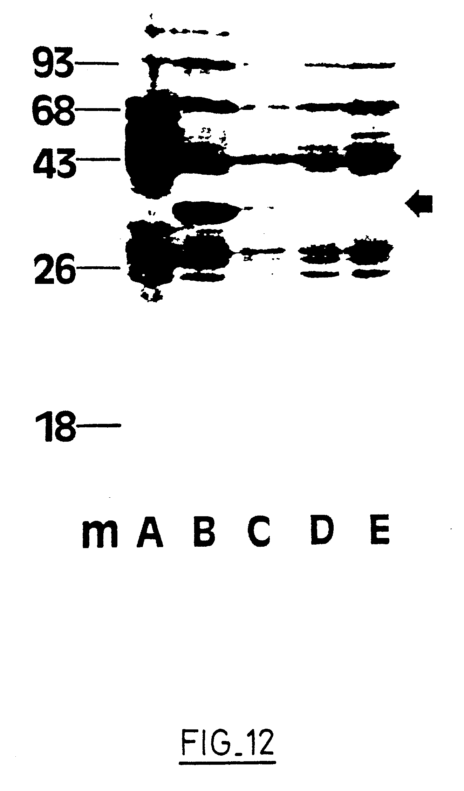

La souche TGE900/pRG123 est mise en croissance sur un milieu enrichi à 28,5°C jusqu'à une densité optique de 0,1 à 660 nm. On ajoute de la L-méthionine $S_{35}$ avec une activité de 5uCi.ml$^{-1}$ et des aliquots sont mis en incubation pendant 5 heures à différentes températures. Les cellules sont concentrées par centrifugation et des échantillons soumis à une électrophorèse sur gel de polyacrylamide à 10 % en présence de SDS, comme cela est décrit par Laemmli (1970). Les bandes radioactives sont révélées par fluorographie (Laskey & Mills, 1975). Les résultats de cette fluorographie sont représentés à la figure 12. Les températures d'incubation sont les suivantes:

A - 28,5°C
B - 34 5°C
C - 37°C
D - 39°C
E - 42°C.

La position des marqueurs de poids moléculaire est indiquée dans la colonne m, les poids moléculaires étant indiqués en kD. La flèche indique la position de la glycoprotéine de la rage et l'on constate une forte influence de la température d'incubation sur la production de cette protéine, la température optimum étant de 34,5°C.

### Reférences

1. Ish-Horowitz D. et Burke J.F., Nucl. Acids Res. 9, 2989 - 2998 (1981).
2. Panayotatos N. et Trong K. Nucl. Acids Res. 9, 5679 - 5688 (1981).
3. Hoopes B.C. et McClure R.R., Nucl. Acids Res. 9, 5493 - 5504 (1981).
4. Dagert M. et Ehrlich S.D., Gene, 23 - 28 (1979).
5. Vieira J. et Messing J., Gene 19, 259 - 268 (1982).
6. Shimatake H. et Rosenberg M., Nature, 292, 128 - 132, (1981).
7. Burnette W.N., Analytical Biochem. 112, 195 - 203 (1981).
8. Laemmli U.K., Nature 227, 680 - 685 (1970).
9. Laskey R.A. & Mills A.D., Eur. J. Biochem., 56, 335 - 341 (1975).

### Revendications

1. Bactérie exprimant une protéine antigénique de la rage, caractérisée en ce que ladite bactérie a été transformée ou transfectée par un vecteur d'expression qui comporte:
- la séquence d'ADN suivante:
AGA GGC CTA TAT AAG TCT TTA AAA GGA GCA TGC AAA CTC AAG TTA
TGT GGA GTT CTA GGA CTT AGA CTT ATG GAT GGA ACA TGG GTC GCG
- un promoteur de l'expression de cette séquence dans ladite bactérie.
2. Bactérie selon la revendication 1, caractérisée en ce qu'elle comporte tout ou partie de l'ADN d'un phage M13.
3. Bactérie selon l'une des revendications 1 et 2, caractérisée en ce que le promoteur de l'expression de la séquence d'ADN est un promoteur de bactériophage lambda.
4. Bactérie selon l'une des revendications 1 à 3, caractérisée en ce qu'il s'agit d'un plasmide bactérien.
5. Bactérie selon la revendication 4, caractérisée en ce qu'elle comporte au moins:
- l'origine de réplication d'un plasmide bactérien,

- une région d'initiation de la traduction,
- un promoteur du bactériophage lambda $P_L$, $P_R$ ou $P'_R$.

6. Bactérie selon la revendication 5, caractérisée en ce que la région d'initiation de la traduction est lambdacllrbs.

7. Bactérie selon l'une des revendications 5 et 6, caractérisée en ce que le promoteur utilisé est le promoteur $P_L$.

8. Bactérie selon l'une des revendications 5 à 7, caractérisée en ce qu'elle comporte, en outre, une fonction d'antiterminaison de transcription.

9. Bactérie selon l'une des revendications 5 à 8, caractérisée en ce qu'elle comporte l'origine de réplication de pBR322.

10. Bactérie selon l'une des revendications 5 à 9, caractérisée en ce qu'elle comporte un gène codant pour la résistance à un antibiotique.

11. Bactérie selon l'une des revendications 5 à 10, caractérisée en ce que le promoteur est contrôlé par un répresseur.

12. Bactérie seion la revendication 11, caractérisée en ce que la séquence de répression est induite par une élévation de température.

13. Bactérie seion l'une des revendications 1 à 12, caractérisée en ce qu'il s'agit d'une souche de E. coli ou de B. subtilis.

14. Procédé de préparation d'une protéine antigénique de la rage, caractérisé en ce qu'on cultive sur un milieu approprié une bactérie selon l'une des revendications 1 à 13.

15. Procédé selon la revendication 14, caractérisé en ce que la bacterie est un E. coli ou un B. subtilis.


## Patentansprüche

1. Bakterium, das ein Tollwut-Antigenprotein ausscheidet, dadurch gekennzeichnet, daß das Bakterium durch ein Segment einer Formel umgewandelt oder verändert wurde, welches aufweist:
- die folgende ADN-Sequenz:

AGA GGC CTA TAT AAG TCT TTA AAA GGA GCA TGC AAA CTC AAG TTA
TGT GGA GTT CTA GGA CTT AGA CTT ATG GAT GGA ACA TGG GTC GCG
- einen Promotor der Formel dieser Sequenz in dem Bakterium.

2. Bakterium nach Anspruch 1, dadurch gekennzeichnet, daß es die ganze oder einen Teil der ADN-Sequenz von einem M13 Phagen enthält.

3. Bakterium nach einem der Ansprüche 1 und 2, dadurch gekennzeichnet, daß der Promotor der Formel der ADN-Sequenz ein Promotor der Lambda-Bakteriophage ist.

4. Bakterium nach einem der Ansprüche 1 bis 3, dadurch gekennzeichnet, daß es sich um ein bakterielles Plasmid handelt.

5. Bakterium nach Anspruch 4, dadurch gekennzeichnet, daß es mindestens enthält:
- den Sitz der Neusynthese eines bakteriellen Plasmides,
- einen Bereich der Einleitung der Umwandlung,
- einen Promotor der Lambda-Bakteriophage $P_L$, $P_R$ oder $P'_R$.

6. Bakterium nach Anspruch 5, dadurch gekennzeichnet, daß der Bereich der Einleitung der Umwandlung Lambdacllrbs ist.

7. Bakterium nach einem der Ansprüche 5 und 6, dadurch gekennzeichnet, daß der verwendete Promotor der Promotor $P_L$ ist.

8. Bakterium nach einem der Ansprüche 5 bis 7, dadurch gekennzeichnet, daß es weiterhin eine Funktion aufweist, die die Beendigung der Umwandlung verhindert.

9. Bakterium nach einem der Ansprüche 5 bis 8, dadurch gekennzeichnet, daß es den Sitz der Neusynthese von pBR322 aufweist.

10. Bakterium nach einem der Ansprüche 5 bis 9, dadurch gekennzeichnet, daß es ein Gen aufweist, das für den Widerstand gegenüber einem Antibiotikum kodiert ist.

11. Bakterium nach einem der Ansprüche 5 bis 10, dadurch gekennzeichnet, daß der Promotor durch einen Blocker gesteuert wird.

12. Bakterium nach Anspruch 11, dadurch gekennzeichnet, daß die Sequenz der Blockierung durch eine Temperaturerhöhung induziert wird.

13. Bakterium nach einem der Ansprüche 1 bis 12, dadurch gekennzeichnet, daß es sich um einen Stamm von E. coli oder von B. subtilis handelt.

14. Verfahren zur Herstellung eines Tollwut-Antigenproteins, dadurch gekennzeichnet, daß man in einem geeigneten Milieu ein Bakterium nach einem der Ansprüche 1 bis 13 kultiviert.

15. Verfahren nach Anspruch 14, dadurch gekennzeichnet, daß das Bakterium ein E. coli oder ein B. subtilis Bakterium ist.

**Claims**

1. Bacterium expressing an antigenic protein of rabies, characterized in that the said bacterium has been transformed or transfected by an expression vector which contains:
- the following DNA sequence:
AGA GGC CTA TAT AAG TCT TTA AAA GGA GCA TGC AAA CTC AAG
TTA TGT GGA GTT CTA GGA CTT AGA CTT ATG GAT GGA ACA TGG GTC GCG
- a promoter for the expression of this sequence in the said bacterium.

2. Bacterium according to Claim 1, characterized in that it contains all or part of the DNA of an M13 phage.

3. Bacterium according to one of Claims 1 and 2, characterized in that the promoter for the expression of the DNA sequence is a bacteriophage lambda promoter.

4. Bacterium according to one of Claims 1 to 3, characterized in that a bacterial plasmid is involved.

5. Bacterium according to Claim 4, characterized in that it contains at least:
- the origin of replication of a bacterial plasmid,
- a translation initiation region,
- a bacteriophage lambda promoter $P_L$, $P_R$ or $P'_R$.

6. Bacterium according to Claim 5, characterized in that the translation initiation region is lambdacIIrbs.

7. Bacterium according to one of Claims 5 and 6, characterized in that the promoter used is the $P_L$ promoter.

8. Bacterium according to one of Claims 5 to 7, characterized in that it contains, in addition, a transcription antitermination function.

9. Bacterium according to one of Claims 5 to 8, characterized in that it contains the origin of replication of pBR322.

10. Bacterium according to one of Claims 5 to 9, characterized in that it contains a gene coding for resistance to an antibiotic.

11. Bacterium according to one of Claims 5 to 10, characterized in that the promoter is controlled by a repressor.

12. Bacterium according to Claim 11, characterized in that the repression sequence is induced by a temperature rise.

13. Bacterium according to one of Claims 1 to 12, characterized in that it is a strain of E. coli or of B. subtilis.

14. Method for preparing an antigenic protein of rabies, characterized in that a bacterium according to one of Claims 1 to 13 is cultured on a suitable medium.

15. Method according to Claim 14, characterized in that the bacterium is an E. coli or a B. subtilis.

PstI                                    −19                                                          61                                            1

CTG CAG GGG GGG GGG GGG GGA GGA AAG ATG GTT CCT CAG GCT CTC CTG TTT GTA CCC CTT CTG GTT TTT CCA TTG TGT TTT GGG AAA TTC
                                        Met Val Pro Gln Ala Leu Leu Phe Val Pro Leu Leu Val Phe Pro Leu Cys Phe Gly Lys Phe

91                              HindIII      121        HgiJII                               PvuII
CCT ATT TAC ACG ATA CTA GAC AAG CTT GGT CCC TGG TCC CCG ATT GAC ATA CAT CAC CTC TCT TGC CCA AAC AAT TTG GTA GTG GAG GAC
Pro Ile Tyr Thr Ile Leu Asp Lys Leu Gly Pro Trp Ser Pro Ile Asp Ile His His Leu Ser Cys Pro Asn Asn Leu Val Val Glu Asp

181                                              211
GAA GGA TGC ACC AAC CTG TCA GGG TTC TCC TAC ATG GAA CTT AAA GTT GGA TAC ATC TTA GCC ATA AAA ATG AAC GGG TTC ACT TGC ACA
Glu Gly Cys Thr Asn Leu Ser Gly Phe Ser Tyr Met Glu Leu Lys Val Gly Tyr Ile Leu Ala Ile Lys Met Asn Gly Phe Thr Cys Thr

271                                              301                                    331
GGC GTT GTG ACG GAG GCT GAA ACC TAC ACT AAC TTC GTT GGT TAT GTC ACA ACC ACG TTC AAA AGA AAG CAT TTC CGC CCA ACA CCA GAT
Gly Val Val Thr Glu Ala Glu Thr Tyr Thr Asn Phe Val Gly Tyr Val Thr Thr Thr Phe Lys Arg Lys His Phe Arg Pro Thr Pro Asp

AvaIII                                   HpaII BstEII                            421
AGA GCC GCG TAC AAC TGG AAG ATG GTC ATT GAC CCC AGA TAT GAA GAG TCT CTA CAC AAT CCG TAC CCT GAC TAC CGC TGG CTT
Arg Ala Ala Tyr Asn Trp Lys Met Ala Gly Asp Pro Arg Tyr Glu Glu Ser Leu His Asn Pro Tyr Pro Asp Tyr Arg Trp Leu

AsuII                                        481                                   511                          XhoI AvaI
ACT GTA AAA ACC ACC AAG GAG TCT CTC GTT ATC ATA TCT CCA AGT GTA GCA GAT TTG GAC CCA TAT GAC AGA TCC CTT CAC
Arg Thr Val Lys Thr Thr Lys Glu Ser Leu Val Ile Ile Ser Pro Ser Val Ala Asp Leu Asp Pro Tyr Asp Arg Ser Leu His Ser His

541                                         571                                    601                         AvaI
GTC TTC CCT AGC GGG AAG TGC TCA GGA GTA GCG GTG TCT TCT ACC TAC TGC TCC ACT AAC CAC GAT TAC ACC ATT TGG ATG       AAT
Val Phe Pro Ser Gly Lys Cys Ser Gly Val Ala Val Ser Ser Thr Tyr Cys Ser Thr Asn His Asp Tyr Thr Ile Trp Met Pro Glu Asn

631                                   661                                    691
CCG AGA CTA GGG ATG TCT TGT GAC ATT TTT ACC AAT AGT AGA GGG AAG AGA GCA TCC AAA GGG AGT GAG ACT TGC GGC TTT GTA GAT GAA
Pro Arg Leu Gly Met Ser Cys Asp Ile Phe Thr Asn Ser Arg Gly Lys Arg Ala Ser Lys Gly Ser Glu Thr Cys Gly Phe Val Asp Glu

721 StuI HaeI          AhaIII      SphI                                    781                          NruI
AGA GGC CTG TAT AAG TCT TTG AGA GGA GCA TTT AAA CTC AAG TTA TGT GGA GTT CTA GGA CTT AGA CTT ATG GAT GGA ACA TGG GTT CAC
Arg Gly Leu Tyr Lys Ser Leu Lys Gly Ala Cys Lys Leu Lys Leu Cys Gly Val Leu Gly Leu Arg Leu Met Asp Gly Thr Trp Val His

FIG. 1a

EP 0 094 887 B1

811                                    841                                                      871
ATG CAA ACA TCA AAT GAA ACC AAA TGG TGC CCT CCT GAT CAG TTG GTG AAC CTG CAC GAC TTT CGC TCA GAC GAA ATT GAG CAC CTT GTT
Met Gln Thr Ser Asn Glu Thr Lys Trp Cys Pro Pro Asp Gln Leu Val Asn Leu His Asp Phe Arg Ser Asp Glu Ile Glu His Leu Val

BgaI  961                                                                          AcyI
901                                    931
GTA GAG GAG TTG GTC AGG AAG AGA GAG GAG TGT CTG GAT GCA CTA GAG TCC ATC ATG ACA ACC AAG TCA GTG AGT TTC AGA AGT CTC AGT
Val Glu Glu Leu Val Arg Lys Arg Glu Glu Cys Leu Asp Ala Leu Glu Ser Ile Met Thr Thr Lys Ser Val Ser Phe Arg Arg Leu Ser

991                                    1021                                  1051
CAT TTA AGA AAA CTT GTC CCT GGG TTT GGA AAA GCA TAT ACC ATA TTC AAC AAG ACC TTG ATG GAA GCC GAT GCT CAC TAC AAG TCA GTC
His Leu Arg Lys Leu Val Pro Gly Phe Gly Lys Ala Tyr Thr Ile Phe Asn Lys Thr Leu Met Glu Ala Asp Ala His Tyr Lys Ser Val

1081                                   1111                                 1141
AGA ACT TGG AAT GAG ATC CTC CCT TCA AAA GGG TGT TTA AGA GTT GGG GGG AGG TGT CAT CCT CAT GTG AAC GGG GTG TTT TTC AAT GGT
Arg Thr Trp Asn Glu Ile Leu Pro Ser Lys Gly Cys Leu Arg Val Gly Gly Arg Cys His Pro His Val Asn Gly Val Phe Phe Asn Gly

1171                                   1201                                 1231
ATA ATA TTA GGA CCT GAC GGC AAT GTC TTA ATC CCA GAG ATG CAA TCA TCC CTC CTC CAG CAA CAT ATG GAG TTG TTG GAA TCC TCG GTT
Ile Ile Leu Gly Pro Asp Gly Asn Val Leu Ile Pro Glu Met Gln Ser Ser Leu Leu Gln Gln His Met Glu Leu Leu Glu Ser Ser Val

AccI
1261                                                                       1321
ATC CCC CTT GTG CAC CCC CTG GCA GAC CCG TCT ACC GTT TTC AAG GAC GGT GAC GAG GCT GAG GAT TTT GTT GAA GTT CAC CTT CCC GAT
Ile Pro Leu Val His Pro Leu Ala Asp Pro Ser Thr Val Phe Lys Asp Gly Asp Glu Ala Glu Asp Phe Val Glu Val His Leu Pro Asp

HindII 1381                                                 1411              ApaI HgiJII
1351
GTG CAC AAT CAG GTC TCA GGA GTT GAC TTG GGT CTC CCG AAC TGG GGG AAG TAT GTA TTA CTG AGT GCA GGG GCC CTG ACT GCC TTG ATG
Val His Asn Gln Val Ser Gly Val Asp Leu Gly Leu Pro Asn Trp Gly Lys Tyr Val Leu Leu Ser Ala Gly Ala Leu Thr Ala Leu Met

ClaI                                                     1501
1441                                   1471
TTG ATA ATT TTC CTG ATG ACA TGT TGT AGA AGA GTC AAT CGA TCA GAA CCT ACG CAA CAC AAT CTC AGA GGG ACA GGG AGG GAG GTG TCA
Leu Ile Ile Phe Leu Met Thr Cys Cys Arg Arg Val Asn Arg Ser Glu Pro Thr Gln His Asn Leu Arg Gly Thr Gly Arg Glu Val Ser

1531                                   1561                       1591   505
GTC ACT CCC CAA AGC GGG AAG ATC ATA TCT TCA TGG GAA TCA CAC AAG AGT GGG GGT GAG ACC AGA TTG TGA GGA CTG GCC GTC CTT TCA
Val Thr Pro Gln Ser Gly Lys Ile Ile Ser Ser Trp Glu Ser His Lys Ser Gly Gly Glu Thr Arg Leu ***

PstI
1621                                   1651                                 1681
ACG ATC CAA GTC CTG AAG ATC ACC TCC CCT TGG GGG GTT CTT TTT AAA AAA AAA AAA AAA AAA AAA AAA ACC CCC CCC CCC CCC CTG CA
1710

# FIG_1b

FIG. 2

FIG. 3

EP 0 094 887 B1

M13MF7

MET THR MET ILE THR ASN SER PRO ASP PRO SER THR CYS ARG SER THR ASP PRO GLY ASN SER LEU ALA    + BLEU
ATG ACC ATG ATT ACG AAT TCC CCG GAT CCG TCG ACC TGC AGG TCG ACG GAT CCG GGG AAT TCA CTG GCC

ECO RI        BAM HI   SAL I    PST I    SAL I   BAM HI       ECO RI


M13MP701

MET THR MET ILE THR ASN SER PRO ASP PRO SER THR CYS SER ASN SER LEU ALA    + BLEU
ATG ACC ATG ATT ACG AAT TCC CCG GAT CCG TCG ACC TGC AGC AAT TCA CTG GCC

ECO RI        BAM HI  SAL I   PST I


M13TG102
(M13TG103A)

MET THR MET ILE THR ASN SER PRO ASP PRO SER PRO SER LEU ALA THR CYS SER ASN SER LEU ALA    + BLEU
ATG ACC ATG ATT ACG AAT TCC CCG GAT CCG TCG CCA AGC TTG GCG ACC TGC AGC AAT TCA CTG GCC

ECORI       BAMHI       HINDIII       PSTI


M13TG110
(M13TG114A)

                          ECORI      BAMHI       HINDIII         PSTI
MET THR MET ILE THR ASN SER PRO ASP PRO SER LYS LEU GLY PRO ALA ALA ILE HIS    − BLANC
ATG ACC ATG ATT ACG AAT TCC CCG GAT CCG TCC AAG CTT GGA CCT GCA GCA ATT CAC

ECORI       BAMHI       HINDIII       PST1


M13TG111

MET THR MET ILE THR ASN SER PRO ASP ARG SER VAL ALA LYS LEU GLY ASP LEU GLN GLN PHE    − BLANC
ATG ACC ATG ATT ACG AAT TCC CCG GAT CGA TCC GTC GCC AAG CTT GGC GAC CTG CAG CAA TTC

ECO RI        CLA I         HINDIII       PST I


M13TG112

MET THR MET ILE THR ASN SER PRO ASP ARG SER VAL GLN ALA VAL ARG PRO ALA ALA ILE HIS    − BLANC
ATG ACC ATG ATT ACG AAT TCC CCG GAT CGA TCC GTC CAA GCT TGG CGA CCT GCA GCA ATT CAC

ECO RI        CLA I         HINDIII       PST I


# FIG_4a

FIG_4b

EP 0 094 887 B1

# FIG. 5

FIG. 6

LAC I'

PvuII

NarI/HaeII

EcoRI

BamHI

Plac

C1

5' fmet   CII RBS

LAC Z'

BamHI

HaeII

PvuII

HindIII

PstI

PvuI

BglII

ClaI

## FIG_7

17

FIG. 8

FIG_9

A   DNA Fragment Present in M13tgRG7 and Its Translation Product
------------------------------------------------------------------------

                                                          HindIII------//------409 Amino Acids---------->

                                                                              Apal/Hgi JII
                                    HindII                                    --------
                                    ------
CTT CCC GAT GTG CAC AAT CAG GTC TCA GGA GTT GAC TTG GGT CTC CCG AAC TGG GGG AAG TAT GTA TTA CTG AGT GCA GGG GCC CTG ACT
Leu Pro Asp Val His Asn Gln Val Ser Gly Val Asp Leu Gly Leu Pro Asn Trp Gly LysTyr Val Leu Leu Ser Ala Gly Ala Leu Thr
                                            420                                       I----------Transmembrane-Hydrophobic----


GCC TTG ATG TTG ATA ATT TTC CTG ATG ACA TGT TGT AGA AGA GTC AAT CGA TCA GAA CCT ACG CAA CAC AAT CTC AGA GGG ACA GGG AGG
Ala Leu Met Leu Ile Ile Phe Leu Met Thr Cys CysIArg Arg Val Asn Arg Ser Glu Pro Thr Gln His Asn Leu Arg Gly Thr Gly Arg
----Zone-----------------------------------------I


AAG GTG TCA GTC ACT CCC CAA AGC GGG AAG ATC ATA TCT TCA TGG GAA TCA CAC AAG AGT GGG GGT GAG ACC AGA CTG TGA
Glu Val Ser Val Thr Pro Gln Ser Gly Lys Ile Ile Ser Ser Trp Glu Ser His Lys Ser Gly Gly Glu Thr Arg Leu ***
                                                                              495 Amino acids from
                                                                              HindIII site


B   DNA Fragment Present in M13tgRG151 and Its Translation Product
------------------------------------------------------------------------
(Cleavage of M13tgRG7 with HindII and Insertion of a decameric BamHI linker)

                                                          HindIII------//------409 Amino Acids---------->

                                                                              Apal/
                                                                              --
CTT CCC GAT GTG CAC AAT CAG GTC TCA GGA GTT ccg gat ccg gGA CTT GGG TCT CCC GAA CTG GGG GAA GTA TGT ATT ACT GAG TGC AGG
Leu Pro Asp Val His Asn Gln Val Ser Gly Val Pro Asp Pro Gly Leu Gly Ser Pro Glu Leu Gly Glu Val Cys Ile Thr Glu Cys Arg
                                            420 ============
HgiJII                                           linker
------
GGC CCT GAC TGC CTT GAT GTT GAT AAT TTT CCT GAT GAC ATG TTG TAG AAG AGT CAA TCG ATC AGA ACC TAC GCA ACA CAA TCT CAG AGG
Gly Pro Asp Cys Leu Asp Val Asp Asn Phe Pro Asp Asp Met Trp ***
                                            464 amino acids from
                                            HindIII site


GAC AGG GAG GGA GGT GTC AGT CAC TCC CCA AAG CGG GAA GAT CAT ATC TTC ATG GGA ATC ACA CAA GAG TGG GGG TGA GAC CAG ACT GTG

## FIG_10

EP 0 094 887 B1

FIG. 11

FIG. 12

m A B C D E

93—
68—
43—
26—
18—